(19) Europäisches Patentamt
European Patent Office
Office européen des brevets

(11) Publication number: 0 308 084 A1

# (12) EUROPEAN PATENT APPLICATION

(21) Application number: 88307790.1

(22) Date of filing: 23.08.88

(51) Int. Cl.4: C07D 213/803 , C07D 471/04 , C07D 213/80 , C07D 213/82 , C07D 213/85 , C07D 491/048 , //C07D211/94

(30) Priority: 18.09.87 GB 8721986

(43) Date of publication of application: 22.03.89 Bulletin 89/12

(84) Designated Contracting States: BE CH DE ES FR GB IT LI NL SE

(71) Applicant: IMPERIAL CHEMICAL INDUSTRIES PLC
Imperial Chemical House Millbank
London SW1P 3JF(GB)

(72) Inventor: Crosby, John
12 Oakwood Court Bow Lane
Bowden Altrincham(GB)
Inventor: Fielden, Jan Michael
19 Ashington Drive, off Ainsworth Road
Bury Lancashire(GB)

(74) Representative: Pugsley, Roger Graham et al
IMPERIAL CHEMICAL INDUSTRIES PLC Legal Department: Patents PO Box 6 Bessemer Road
Welwyn Garden City Herts, AL7 1HD(GB)

## (54) Pyridine derivative.

(57) A process which comprises reacting an oxime of the formula:

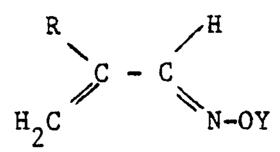

I

wherein
R is H, alkyl, alkenyl, alkoxy, alkylthio, phenyl, phenoxy, halogen: and
Y is H or a group capable of substitution at the oxygen atom of an oxime;
with a compound of the formula:

EP 0 308 084 A1

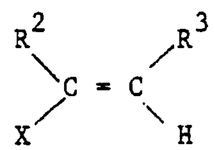

II

wherein
$R^2$ & $R^3$ are each independently H, COZ or CN, provided that they are not both H: or
$R^2$ & $R^3$ together are -CO-O-CO- or -CO-$NR^4$-CO-
Z is $OR^4$ or $NR^4R^5$;
$R^4$ & $R^5$ are each independently H or groups capable of forming an ester or amide with a carboxylic acid: or
$R^4$ & $R^5$ together with the N atom form a aliphatic or aromatic heterocycle;
and X is halogen;
is useful for the preparation of intermediates for important herbicides.

# Pyridine Derivative

This invention relates to a process for the preparation of pyridine derivatives and to certain novel pyridine derivatives.

According to the present invention there is provided a process which comprises reacting an oxime of the formula:

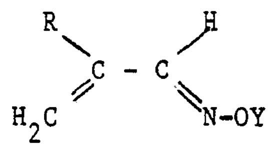

I

wherein

R is H, alkyl, alkenyl, alkoxy, alkylthio, phenyl, phenoxy, halogen; and

Y is H or a group capable of substitution at the oxygen atom of an oxime;

with a compound of the formula:

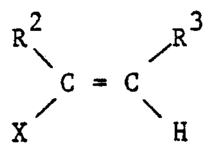

II

wherein

$R^2$ & $R^3$ are each independently H, COZ or CN, provided that they are not both H; or

$R^2$ & $R^3$ together are -CO-O-CO- or -CO-$NR^4$-CO-

Z is $OR^4$ or $NR^4R^5$;

$R^4$ & $R^5$ are each independently H or groups capable of forming an ester or amide with a carboxylic acid; or

$R^4$ & $R^5$ together with the N atom form a aliphatic or aromatic heterocycle;

and X is halogen.

In the oxime of Formula I the group represented by Y is preferably alkyl, alkenyl, alkyl-CO- or alkyl-$SO_2$- especially those in which the alkyl or alkenyl group contains up to four carbon atoms, or aryl, aryl-CO- or aryl-$SO_2$-, in which the aryl group is preferably phenyl. It is preferred that Y is H.

The group represented by R may be unsubstituted or substituted. Suitable substituents for the alkyl and alkenyl groups in R are hydroxy, halogen, $C1_{1-4}$-alkoxy, phenyl, phenoxy, cyano, carboxyl, and $C_{1-4}$-alkoxycarbonyl. Suitable substituents for the phenyl groups in R are halogen, $C_{1-4}$-alkyl, cyano, $C_{1-4}$-alkoxy, $C_{2-4}$-alkenyl, $C_{1-4}$-haloalkyl and $C_{1-4}$-cyanoalkyl. Where R is or contains an alkyl or alkenyl group this may be linear or branched and preferably contains up to 6 carbon atoms, more preferably up to 4 carbon atoms. It is especially preferred that R is ethyl.

In the compound of Formula II it is preferred that $R^2$ and $R^3$ are carboxylic acid groups or precursors thereof such as carbonamide or cyano groups or that $R^2$ and $R^3$ together form a cyclic anhydride or amide, -CO-O-CO- or -CO-$NR^4$-CO-, which may be hydrolysed to form adjacent carboxylic acid groups. It is especially preferred that the compound of Formula II is in the form of a half amide or a cyclic amide, in which $R^2$ and $R^3$ are COOH and/or $CONHR^4$ or $R^2$ and $R^3$ together form a group -CO-$NR^4$-CO-.

The groups represented by $R^4$ and $R^5$ are conveniently optionally substituted alkyl, aryl or aralkyl, such as $C_{1-4}$-alkyl, phenyl or benzyl. Where $R^4$ and $R^5$ form part of a heterocycle this is preferably monocyclic such as piperidine, piperazine, morpholine or pyridine. Suitable substituents include -$CONH_2$, CN and COOH.

Preferred compounds of Formula II are:

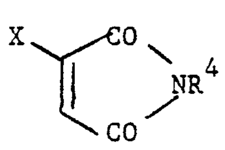

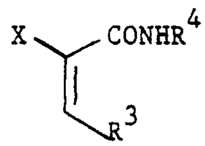

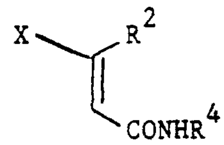

including individual enantiomers when the group $R^4$ contains a chiral centre. especially where $R^4$ is

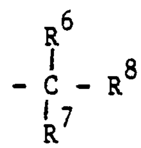

in which

$R^6$ is H or linear or branched $C_{1-5}$-alkyl;

$R^7$ is H, linear or branched $C_{1-5}$-alkyl or $C_{3-6}$-cycloalkyl; or

$R^6$ & $R^7$ together with the carbon atom to which they are attached are $C_{3-5}$-cycloalkyl, optionally substituted with alkyl groups;

and $R^8$ is CN or $CONH_2$.

It is also preferred that X is chlorine or bromine.

The process of the invention may be represented graphically by the following equations:

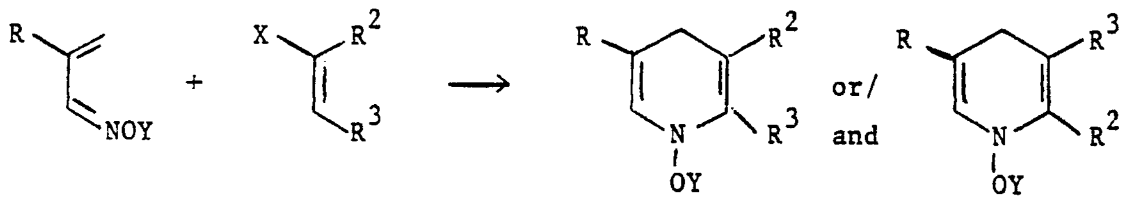
or/ and

I II IIIa IIIb

where the compounds of formula III are the primary reaction products from which, by further elimination of HOY, compounds of Formula IV are produced:

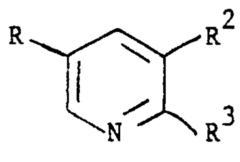

and/or

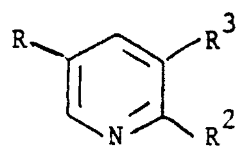

IVa IVb

The orientation of substituents in the compounds of Formulae IV and III is dependent upon the particular combination of X, $R^2$ and $R^3$.

The reaction is preferably carried out in an inert medium, optionally in the presence of an inorganic base and, where it is not intended to isolate the compound of Formula III, at a sufficient temperature to ensure the in situ conversion of this into the compound of Formula IV. Where Y = H and water is being eliminated a particularly convenient one-pot process may be carried out in which the compounds of Formulae I and II react first to give the dihydropyridine of Formula III which is dehydrated in situ to the pyridine of Formula IV. By suitable choice of solvent, e.g. toluene, the eliminated water may be removed azeotropically as it is formed.

The process is useful for the preparation of pyridine and hydropyridine derivatives. especially 5-substituted pyridines having two carboxylic acid groups, or precursors thereof. in the 2 and 3 positions on the ring. Such compounds are intermediates in the preparation of certain pyridines having useful herbicidal properties.

Depending on the reaction conditions employed and the nature of the substituents. $R^2$ and $R^3$. it is possible to isolate a novel intermediate of the general formula III:

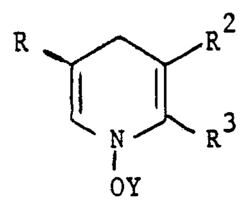

III

wherein Y, R, $R^2$, $R^3$ are as previously defined. However, the compound in which Y is H readily de-hydrates to form a pyridine. In the compound of Formula III it is preferred that R is $C_{1-4}$-alkyl or $C_{2-4}$-alkenyl and especially ethyl.

It is preferred that $R^2$ and $R^3$ are carboxylic acid groups or precursors thereof such as carbonamide or cyano groups or $R^2$ and $R^3$ together form a cyclic anhydride or amide. -CO-O-CO- or -CO-$NR^4$-CO-, which may be hydrolysed to a pyridine dicarboxylic acid or may be used in its own right (see Scheme A).

<u>Scheme A</u>

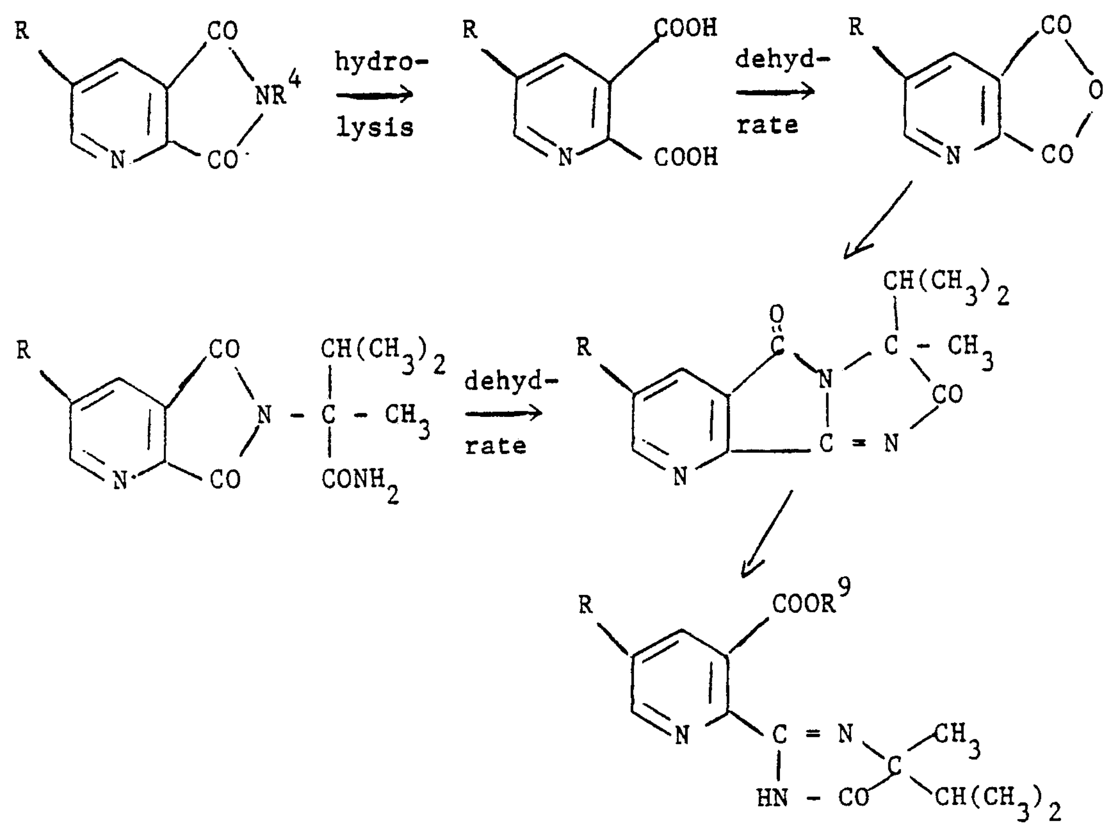


The pyridine dicarboxylic acids and their precursors, especially those with a 5-ethyl substituent, are useful intermediates for the preparation of pyridine-based herbicides.

Especially preferred compounds of Formula III are:

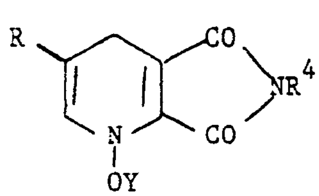
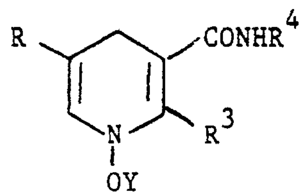
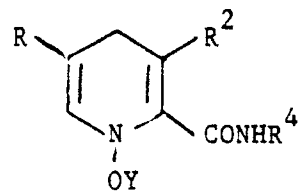

including the individual enantiomers when the group $R^4$ contains a chiral centre, where $R^4$ is

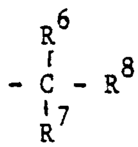

in which
$R^6$ is H or linear or branched $C_{1-5}$-alkyl;
$R^7$ is H, linear or branched $C_{1-5}$-alkyl or $C_{3-5}$-cycloalkyl; or
$R^6$ & $R^7$ together with the carbon atom to which they are attached are $C_{3-5}$-cycloalkyl, optionally substituted with alkyl groups;
and $R^8$ is CN or $CONH_2$.

The starting material of Formula I is conveniently prepared by reaction of an aldehyde of the formula, $R\text{-}CH_2\text{-}CHO$, with formaldehyde to produce a substitued acrolein of the formula, $R\text{-}C(CHO){=}CH_2$ as for example described by Marvel et al, JACS 70 1694 and then with an optionally substituted hydroxylamine of the formula, $NH_2OY$. The starting material of Formula II is conveniently prepared by the halogenation of a maleic acid or derivative thereof, followed by dehydrohalogenation:

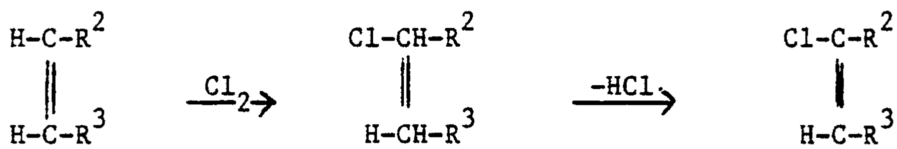


and compounds with different values of $R^2$ and $R^3$ can be obtained therefrom in a known manner.

The invention is illustrated by the following Examples in which all parts and percentages are by weight unless otherwise indicated.

Example 1

Mono-chloro-N-phenylmaleimide (363.1g; 1.75g.mole) and calcium carbonate (87.5g; 0.875g.mole) were stirred at reflux in toluene (650g; 7.07g.moles) whilst 2-ethyl acrolein oxime (190.6g; 1.925g.mole) was added over 6 to 7 hours with concurrent removal of generated water by azeotropic distillation. The reaction mass was stirred at reflux for a further 4 to 5 hours to complete the reaction as judged by gas chromatography (GC). The hot reaction slurry was then screened to remove inorganics and the filtrates cooled to 20 °C. The crystalline product was filtered off, washed with toluene and oven dried at 50 °C under vacuum to obtain 5-ethyl-N-phenylpyridine-2,3-dicarboximide.

The mono-chloro-N-phenylmaleimide was prepared as follows:-

Stage 1 Monochloromaleic anhydride

Maleic anhydride (1666g; 17.0g.moles) and ferric chloride (51.0g; 0.31g.mole) were heated to 105-110 °C and then chlorine (1789g; 25.2g.moles) was passed into the slurry over a period of 25 hours until the level of unreacted maleic anhydride fell to <1% as judged by GC. The reaction rate fell off towards the end of the reaction. Some dehydrohalogenation occurred at 105-110 °C (approx. 20% conversion to monochloromaleic anhydride by the end of the chlorine addition) and further chlorination-dehydrochlorination of this product to alpha-beta-dichloromaleic anhydride occurred (separation of mono and dichloromaleic anhydride is conveniently facilitated by the stage 2 reaction - see below).

The reaction temperature was raised to 180 °C over 3 hours and the slurry stirred at this temperature for 18-20 hours. The reaction was again followed by GC until dehydrochlorination was complete. The slurry was then cooled to 95 °C and the flask set for distillation. Vacuum was applied (10mmHg) and the product (mixture of mono and dichloromaleic anhydride) was distilled off (B.pt.90-105 °C,10mmHg).

Yield of monochloromaleic anhydride = 66.6%.

Stage 2 Monochloro-N-phenylmaleamic acid

The mixed mono and dichloromaleic anhyhdride from Stage 1 (650g at 66.6% = 432.9g at 100% as monochloromaleic anhydride; 3.27gmoles) and toluene (1733g; 18.84gmoles) were heated to 75-85 °C and then aniline (300.8g; 3.27gmoles) (charge based on monochloro maleic anhydride content) was added over a period of 1½ hours. Precipitation of the product occurred during the addition. The reaction slurry was stirred for ½ hour on completion of the aniline addition to ensure complete reaction as judged by HPLC. The reaction mixture was cooled to 15 °C and the precipitated monochloro-N-phenylmaleamic acid filtered off, washed with toluene and then vacuum dried at 50 °C. The dichloromaleic anhydride remains in the toluene filtrates.

Stage 3 Monochloro-N-phenylmaleimide

Monochloro-N-phenylmaleamic acid (451g; 2.0gmoles) from Stage 2 and potassium acetate (107.8g; 1.1gmoles) were heated in acetic anhydride (1224g; 12.0gmoles) over 30 minutes to 50 °C. Initially the slurry went into solution but shortly afterwards a mild exotherm occurred and solid precipitated out. The reaction mixture was stirred at 50-55 °C for one hour, cooled to 30 °C and then drowned into water. The precipitated product was filtered off, washed with water and over dried at 50 °C under vacuum.

The 2-ethylacrolein oxide was prepared as follows:

Stage 1 2-Ethylacrolein

This was made by the method of Marvel JACS 70 1694.

Stage 2 2-Ethylacrolein-oxime

2-Ethylacrolein (210g; 2.5gmoles) from Stage 1 and hydroxylamine hydrochloride (191.1g; 2.75gmoles) previously dissolved in water (250g; 13.89gmoles) were charged to a flask and stirred at 20-25 °C whilst a solution of sodium carbonate (145.75g; 1.38gmoles) in water (375g; 20.8gmoles) was added rapidly over 1 to 5 minutes. An exotherm occurred, raising the temperature to 40 °C. The reaction mixture was stirred at 40 °C until the reaction was complete. The lower aqueous layer was separated off and the organic phase washed with water and then dried over magnesium sulphate to give 2-ethylacrolein-oxime.

Examples 2 - 11

The process of Example 1 was repeated under a variety of conditions; results are summarised in the Table.

Table

| Ex | Solvent | Base | Oxime Addition time(mins) | Reaction Temp. | Reaction Time (hr) | % Yield (isolated) |
|---|---|---|---|---|---|---|
| 2 | Ethanol | - | 20 | reflux | 19 | 39 |
| 3 | Acetonitrile | - | 30 | reflux | 24 | 44.4 |
| 4 | Acetic acid | - | 270 | 80-85 °C | 17 | 15.4 |
| 5 | Toluene | MgO | 1 | reflux | 10 | 37.4 |
| 6 | Toluene | $Na_2CO_3$ | 1 | reflux | 24 | 34.9 |
| 7 | Toluene | - | 200 | reflux | 36 | 17.2 |
| 8 | THF | - | <1 | reflux | 60 | 33.2 |
| 9 | DMA | $CaCO_3$ | <1 | 110-120 °C | 19 | 11.6 |
| 10 | " | - | <1 | 100 °C | 5 | 41.4 |
| 11 | " | $CaCO_3$ | <1 | 90-95 °C | 24 | 16.6 |

THF is tetrahydrofuran. DMA is N,N-dimethylacetamide.

## Example 12

Preparation of 5-ethyl-N-phenylpyridine-2,3-dicarboximide by reaction between 2-ethylacrolein oxime and monochloro-N-phenyl maleimide generated *in situ*.

Monochloro-N-phenylmaleamic acid (11.27g; 0.05gmole) and toluene (26g; 0.28gmole) were heated to reflux. The mixture was maintained at reflux whilst 2-ethylacrolein oxime was added over a period of 3-4 hours. The maleamic acid gradually went into solution as dehydration to the imide occurred. The solution was stirred at reflux for a further 26 hours then screened to remove insolubles. The filtrates were stripped on a rotary evaporator to remove the solvent to yield a crude product (Yield = 17.2%).

Further compounds of Formulae IVa and IVb, with the values of R, $R^2$, and $R^3$ given for the starting materials, can be prepared by reaction, under the conditions of Example 1, of different starting materials of Formulae I and II indicated in the following table, in which the various symbols have the meanings set out above in relation to Formulae I and II.

Table

Starting Materials

| Formula I | | Formula II | | | | |
|---|---|---|---|---|---|---|
| R | Y | $R^2$ | $R^3$ | $R^4$ | $R^5$ | X |
| $C_2H_5$ | $COCH_3$ | $-CO-NR^4-CO-$ | | $C_6H_5$ | - | Cl |
| $C_2H_5$ | $COC_6H_5$ | $-CO-NR^4-CO-$ | | $C_6H_5$ | - | Br |
| $C_2H_5$ | $CH_3$ | $-CO-NR^4-CO-$ | | $CH_3$<br>$-C-CH(CH_3)_2$<br>$CONH_2$ | - | Cl |
| $CH_2CH=CH_2$ | H | $COOR^4$ | $COOR^5$ | $C_2H_5$ | $C_2H_5$ | Cl |
| $C_6H_5$ | H | $CONR^4R^5$ | $CONR^4R^5$ | H | H | Cl |
| $-CH_2C_6H_5$ | H | $-CONR^4CO-$ | | $-CH_2C_6H_5$ | - | Br |
| $C_2H_4OC_2H_5$ | H | $CONR^4R^5$ | H | $-CH_2-CH_2-O-CH_2-CH_2-$ | | Cl |
| $C_2H_4OH$ | H | $CONR^4R^5$ | H | $-CH_2-CH_2-NH-CH_2-CH_2-$ | | Cl |
| $C_2H_4Cl$ | H | $CONR^4R^5$ | H | $-CH_2-CH_2-CH_2-CH_2-CH_2-$ | | Cl |
| Cl | H | CN | CN | - | - | Br |
| $C_2H_5$ | H | $-CO-O-CO-$ | | - | - | Br |

**Claims**

1. A process which comprises reacting an oxime of the formula:

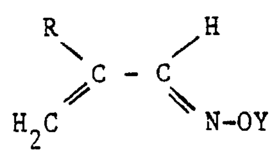

I

wherein
R is H, alkyl, alkenyl, alkoxy, alkylthio, phenyl, phenoxy, halogen; and
Y is H or a group capable of substitution at the oxygen atom of an oxime;
with a compound of the formula:

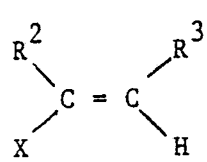

II

wherein
$R^2$ & $R^3$ are each independently H, COZ or CN, provided that they are not both H; or
$R^2$ & $R^3$ together are -CO-O-CO- or $-CO-NR^4-CO-$

Z is $OR^4$ or $NR^4R^5$;
$R^4$ & $R^5$ are each independently H or groups capable of forming an ester or amide with a caboxylic acid: or
$R^4$ & $R^5$ together with the N atom form a aliphatic or aromatic heterocycle:
and X is halogen.

2. A process according to Claim 1 wherein the compound of Formula I. R is $C_{1-4}$-alkyl or phenyl and Y is H.

3. A process according to Claim 2 wherein R is ethyl.

4. A process according to Claim 1 wherein $R^2$ and $R^3$ are selected from H. $-COOR^4$. $-CONR^4R^5$. -CN or $R^2$ and $R^3$ together are -CO-O-CO- or $-CO-NR^4-CO-$ in which $R_4$ is selected from H. $C_{1-4}$-alkyl. phenyl. benzyl and

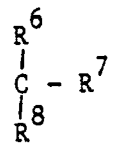

in which $R^6$ is linear or branched $C_{1-5}$-alkyl
$R^7$ is linear or branched $C_{1-5}$-alkyl or $C_{3-5}$-cycloalkyl;
and $R^8$ is CN or $CONH_2$;
$R^5$ is H and X is chlorine or bromine.

European Patent Office

# EUROPEAN SEARCH REPORT

Application number EP 88307790.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2/A3 - 0 161 221 (CIBA-GEIGY)<br>* Claims 1,10,11 *<br>-- | 1,3,4 | C 07 D 213/803<br>C 07 D 471/04<br>C 07 D 213/80<br>C 07 D 213/82<br>C 07 D 213/85<br>C 07 D 491/048<br>//C 07 D 211/94 |
| A | EP - A2/A3 - 0 093 945 (KYOWA HAKKO)<br>* Claim 1; page 4, lines 1-24 *<br>-- | 1,2 | |
| A | US - A - 4 460 776 (WEPPLO)<br>* Claim 1 *<br>---- | 1 | |

TECHNICAL FIELDS SEARCHED (Int. Cl.4)

C 07 D 213/00
C 07 D 471/00
C 07 D 491/00
C 07 D 211/00

The present search report has been drawn up for all claims

| Place of search | Date of completion of the search | Examiner |
|---|---|---|
| VIENNA | 20-12-1988 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82